(19) Europäisches Patentamt
European Patent Office
Office européen des brevets

(11) EP 0 894 504 A2

# (12) EUROPÄISCHE PATENTANMELDUNG

(43) Veröffentlichungstag:
**03.02.1999 Patentblatt 1999/05**

(51) Int. Cl.$^6$: **A61L 29/00**

(21) Anmeldenummer: **98110790.7**

(22) Anmeldetag: **12.06.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.07.1997 DE 19732587**

(71) Anmelder:
**HÜLS AKTIENGESELLSCHAFT**
**45764 Marl (DE)**

(72) Erfinder:
- **Ottersbach, Peter, Dr.**
  **51570 Windeck (DE)**
- **Anders, Christine, Dr.**
  **45721 Haltern (DE)**

## (54) Bioaktive Beschichtung mit reibungsarmer Oberfläche

(57) Die Erfindung betrifft ein Copolymer, welches als Monomere

(A) Maleinsäureanhydrid (Monomer A);
(B) mindestens einen Vinylalkylether, dessen Alkylrest 1 bis 6 Kohlenstoffatome umfaßt (Monomer B);
(C) mindestens ein Carboxyl- und/oder Carboxylatgruppen enthaltendes Vinylmonomer (Monomer C) sowie
(D) mindestens ein Schwefelsäure-, Sulfat-, Sulfonsäure- und/oder Sulfonatgruppen enthaltendes Vinylmonomer (Monomer D)

einpolymerisiert enthält. Die Erfindung betrifft weiterhin die Verwendung der Polymeren für die Herstellung von Erzeugnissen zur Verwendung auf den Gebieten der Nahrungs- und Genußmitteltechnik, Wassertechnik und Biotechnik, der Hygienevorsorge und insbesondere der Medizintechnik und betrifft diese Erzeugnisse als solche.

EP 0 894 504 A2

## Beschreibung

Die Erfindung betrifft Polymere zur Beschichtung von Substraten durch kovalente Anbindung, die aufgrund der Anwesenheit bestimmter funktioneller Gruppen bestimmte bioaktive Eigenschaften zeigen und dazu im wäßrig-feuchten Zustand reibungsarm sind, d.h. einen bemerkenswert niedrigen Reibungskoeffizienten haben. Wichtige bioaktive Eigenschaften der erfindungsgemäßen Polymeren sind ihre bakterienabweisenden Eigenschaften sowie gute Verträglichkeit im Kontakt mit Körperflüssigkeiten und Gewebe. Je nach dem Molverhältnis bestimmter funktioneller Gruppen werden die Oberflächen darüber hinaus zellproliferationshemmend oder zellproliferationsfördernd eingestellt. Die Erfindung betrifft weiterhin Erzeugnisse mit derart beschichteten Oberflächen.

### 1. Stand der Technik und ältere Anmeldungen auf dem Gebiet der bioaktiven und reibungsarmen Polymeren

In der europäischen Patentanmeldung 0 166 998 (entspricht US-A 4 876 126) werden medizinische Instrumente und deren Herstellung beschrieben, wobei u.a. ein wasserlösliches Copolymer (Beschichtungspolymer) aus Maleinsäureanhydrid und Vinylmethylether oder ein nicht notwendigerweise wasserlösliches Derivat eines solchen Copolymers vorzugsweise kovalent, gegebenenfalls aber auch ionisch oder adsorptiv auf einem Polymersubstrat gebunden wird. Die Oberflächen der Beschichtung sollen im befeuchteten (wäßrig-feuchten) Zustand eine gute Gleitfähigkeit zeigen, also einen niedrigen Reibungskoeffizienten haben. Das Copolymer soll, wie die übrigen in der Druckschrift genannten Beschichtungspolymere, eine hydrophile Gruppe enthalten, wobei -OH, $-CONH_2$, -COOH, $-NH_2$, -COO-, $-SO_3^-$ und $NR_3^+$ ausdrücklich genannt werden. Bei kovalenter Bindung wird das Copolymer auf dem Polymersubstrat mittels reaktiver Gruppen kovalent gebunden, von denen Isocyanat-, Amino-, Aldehyd- und Epoxygruppen bevorzugt werden. Wenn die Substratpolymeren von Haus aus keine reaktiven Gruppen enthalten, werden sie mit Verbindungen vorbehandelt, die eine solche Gruppe aufweisen. Polyisocyanate, Polyamine, Polyaldehyde und Polyepoxide werden als geeignete Verbindungen mit reaktiven Gruppen genannt.

In der deutschen Patentanmeldung 197 21 723.0 (O.Z. 5204) wird ein Verfahren zur Verminderung des Reibungswiderstandes von hydrophil beschichteten Polymeroberflächen beschrieben, bei dem man die Beschichtung mit einem Komplexbildner für Schwermetallionen behandelt.

Einer der Gegenstände der deutschen Patentanmeldung 197 23 132.2 (O.Z. 5202) sind wasserunlösliche, bakteriophobe Polymere, erhältlich durch radikalische Copolymerisation von

(c) einem oder mehreren Carboxylgruppen- und/oder Carboxylatgruppen-haltigen, aliphatisch ungesättigten Monomeren oder den entsprechend funktionalisierten Derivaten der Monomeren als Komponente I mit
(d) einem oder mehreren Sulfonsäuregruppen- und/oder Sulfonatgruppen-haltigen, aliphatisch ungesättigten Monomeren oder den entsprechend funktionalisierten Derivaten der Monomeren als Komponente II und
(b) einer Komponente III, die ein weiteres aliphatisch ungesättigtes Monomer oder mehrere weitere aliphatisch ungesättigte Monomere enthält,

wobei gegebenenfalls die entsprechend funktionalisierten Derivate nach der Copolymerisation zumindest an der Oberfläche in Carboxyl- oder Carboxylatgruppen bzw. Sulfonsäure- oder Sulfonatgruppen überführt werden.

Einer der Gegenstände der deutschen Patentanmeldung 197 23 131.4 (O.Z. 5203) sind dieselben Polymeren, wobei es jedoch auf die zellproliferationsinhibierende Wirkung ankommt, die bei, bestimmten Molverhältnissen von Carboxyl- und/oder Carboxylatgruppen zu Sulfonsäure- und/oder Sulfonatgruppen besonders ausgeprägt ist.

Die deutsche Patentanmeldung 197 00 078.9 (O.Z. 5141) betrifft die Polymeren der beiden zuvor genannten Anmeldungen, wobei jedoch auf eine die Zellproliferation begünstigende und gleichzeitig bakterienabweisende Wirkung abgehoben wird, die bei bestimmten Molverhältnissen von Carboxyl- und/oder Carboxylatgruppen zu Sulfonsäureund/oder Sulfonatgruppen gegeben ist.

### 2. Kurzbeschreibung der Erfindung

Es ist eine Aufgabe der Erfindung, neue als Beschichtungsmittel geeignete Polymere bereitzustellen, die zugleich bioaktiv sind und im wäßrig-feuchten Zustand eine reibungsarme Oberfläche haben, wobei die Bioaktivität und der niedrige Reibungswiderstand (bzw. die gute Gleitfähigkeit) auf die Auswahl bestimmter funktioneller Gruppen zurückgehen, ohne daß eine weitere Behandlung erforderlich ist.

Diese Aufgabe wurde gelöst durch ein Copolymer, welches als Monomere

(A) Maleinsäureanhydrid (Monomer A):
(B) mindestens einen Vinylalkylether, dessen Alkylrest 1 bis 6 Kohlenstoffatome umfaßt (Monomer B);
(C) mindestens ein Carboxyl- und/oder Carboxylatgruppen enthaltendes Vinylmonomer (Monomer C) sowie

(D) mindestens ein Schwefelsäure-, Sulfat-, Sulfonsäure- und/oder Sulfonatgruppen enthaltendes Vinylmonomer (Monomer D)

einpolymerisiert enthält.

Die erfindungsgemäßen Polymere enthalten saure Gruppen und/oder davon abgeleitete anionische Gruppen. Gegenionen für die anionischen Gruppen sind in der Regel Alkalimetallionen, insbesondere Natriumionen, oder Ammoniumionen einschließlich quaternärer Ammoniumionen. Das molare Verhältnis von sauren Gruppen zu anionischen Gruppen kann, je nach beabsichtigter Verwendung des Polymers, durch Wahl entsprechender Monomerer bestimmt oder nachträglich durch Behandlung mit Säuren oder Basen verändert werden. Beispielsweise sollten bei medizintechnischen Verwendungen in der Regel keine Schwefelsäure- und Sulfonsäuregruppen vorliegen, die jedoch für Verwendungen auf anderen technischen Gebieten akzeptabel sein können.

Ein anderer Gegenstand der Erfindung ist ein Verfahren zur Herstellung des erfindungsgemäßen Copolymers, bei dem man die Monomeren A, B, C und D radikalisch polymerisiert.

Ein weiterer Gegenstand der Erfindung ist ein Verfahren zum Beschichten von Substraten, bei dem man das Copolymer kovalent auf dem Substrat bindet.

Schließlich sind ein Gegenstand der Erfindung Erzeugnisse, die ganz oder teilweise mit den erfindungsgemäßen Polymeren beschichtet sind und sich Verwendung auf den Gebieten der Nahrungs- und Genußmitteltechnik, Wassertechnik, Biotechnik, Hygienevorsorge und insbesondere der Medizintechnik eignen.

## 3. Vorteile der Erfindung

Die erfindungsgemäßen Polymeren zeigen eine bemerkenswerte Kombination vorteilhafter Eigenschaften und daher eine hervorragende physiologische Verträglichkeit. Sie sind gut blutverträglich und vermindern die Adhäsion und die Vermehrung von Bakterien in einem hohen Maße auch über längere Zeit. Dies gilt u.a. für die Bakterien der Stämme Staphylococcus aureus. Staphylococcus epidermidis. Streptococcus pyogenes, Klebsiella pneumoniae, Pseudomonas aeruginosa und Escherichia coli. Gleichzeitig wird meistens auch die Proliferation von Zellen inhibiert, beispielsweise von Nabelschnurzellen. Die besonderen Bedingungen, unter denen ein Polymer bakterienabweisend, aber zellproliferationsfördernd ist, werden später erläutert. Die Oberflächen der erfindungsgemäßen Polymeren sind frei von migrationsfähigen und/oder extrahierbaren Monomer- und Oligomeranteilen. Unerwünschte Nebenwirkungen, etwa durch freigesetzte körperfremde Stoffe oder durch abgetöte Bakterien, werden nicht beobachtet. Zugleich und nicht zuletzt haben die neuen Polymeren im wäßrig-feuchten Zustand einen bemerkenswert niedrigen Reibungswiderstand.

## 4. Beschreibung der Erfindung

### 4.1 Monomer A

Man verwendet als **Monomer A** das übliche technische reine oder hochreine Maleinsäureanhydrid, wie es in kommerziellen Mengen im Handel erhältlich ist.

### 4.2 Vinylmonomere B

Die Vinylmonomeren B sind Vinylalkylether mit Alkylresten, die 1 bis 6 Kohlenstoffatome, vorteilhaft 1 bis 4 Kohlenstoffatome umfassen. Bevorzugt werden der Vinylethylether und insbesondere der Vinylmethylether.

### 4.3 Monomere c

Bevorzugte Monomere C entsprechen der allgemeinen Formel

$$(C_nH_{2n-q-x})(COOR^1)_x, \qquad (I)$$

in der

- n jeweils unabhängig für eine ganze Zahl von 2 bis einschließlich 6;
- x jeweils unabhängig für 1 oder 2;
- q jeweils unabhängig für 0 oder 2 steht; und
- $R^1$ jeweils unabhängig -H, ein Alkalimetallion oder ein Ammoniumion der Formel $N(R^5)_4^+$ bedeutet, wobei $R^5$ jeweils unabhängig Wasserstoff oder einen organischen Rest, vorzugsweise einen Kohlenwasserstoffrest mit 1 bis 20 Kohlenstoffatomen, bezeichnet.

Von den geeigneten Monomeren C seien beispielsweise genannt: Acrylsäure, Methacrylsäure, 4-Vinylsalicylsäure, Itaconsäure, Vinylessigsäure, Zimtsäure, 4-Vinylbenzoesäure, 2-Vinylbenzoesäure, Sorbinsäure, Kaffeesäure, Methylmaleinsäure, Crotonsäure, Isocrotonsäure, Fumarsäure, Dimethylfumarsäure, Methylfumarsäure, Dihydroxymaleinsäure, Allylessigsäure sowie die Natriumsalze, Ammoniumsalze dieser Säuren mit dem Kation $N(R^5)_4^+$. Beispiele für dieses Kation sind Ammonium, Methylammonium, Dimethylammonium, Trimethylammonium, Tetramethylammonium, Dimethyl-1-n-octylammonium, Dimethyl-1-n-octadecylammonium, Dimethylbenzylammonium und Dimethylanilinium.

Auch vom Benzol abgeleitete Monomere C der allgemeinen Formel

$$(C_6H_{6-b-c-d})[(C_nH_{2n-1-q-x})(COOR^1)_x]_bR^3{}_c(OH)_d \qquad (II)$$

können in dem erfindungsgemäßen Polymer enthalten sein, worin

$R^1$, <u>n</u>, <u>q</u> und <u>x</u> wie zuvor definiert sind;

$R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2-CH_2-N^+(CH_3)_3$, $-PO(O^-)O-CH_2-CH_2-N^+(CH_3)_3$, $-OP(O^-)OCH_2-CH_2-N^+(CH_3)_3$ oder ein Salz, insbesondere ein Alkalisalz, der genannten Gruppen bedeutet;

<u>b</u> für 1, 2, oder 3 steht;

<u>c</u> für 0, 1, 2, oder 3 steht; und

<u>d</u> für 0, 1, 2, oder 3 steht;

mit der Maßgabe, daß $b + c + d \leq 6$, vorteilhaft $\leq 4$ ist.

**4.4 Monomere D**

Bevorzugte Sulfonsäure- und/oder Sulfonatgruppen enthaltende Monomere D entsprechen der allgemeinen Formel

$$(C_nH_{2n-q-x})(SO_3R^1)_x, \qquad (III)$$

in der

<u>n</u>, <u>x</u>, <u>q</u> und $R^1$ die für die Formel I angegebenen Bedeutung haben.

Geeignete Sulfonsäure- und/oder Sulfonatgruppen enthaltende Monomere D der Formel III sind u.a. Natriumallylsulfonat, Natriummethallylsulfonat, Vinylsulfonsäure, Natriumvinylsulfonat, 4-Styrolsulfonsäure, 2-Styrolsulfonsäure, Natrium-2-styrolsulfonat, Natrium-4-styrolsulfonat, Natriumvinyltoluolsulfonat, Dimethyl-1-n-octylammonium-4-styrolsulfonat und Dimethyl-1-n-octylammonium-2-styrolsulfonat

Auch vom Benzol abgeleitete Sulfonsäure- und/oder Sulfonatgruppen enthaltende Monomere der allgemeinen Formel

$$(C_6H_{6-b-c-d})[(C_nH_{2n-1-q-x})(SO_3R^1)_x]_bR^3{}_c(OH)_d \qquad (IV)$$

in der $R^1$, <u>n</u>, <u>q</u> und <u>x</u> sowie $R^3$, <u>b</u>, <u>c</u> und <u>d</u> wie zuvor definiert sind.

eignen sich als Monomere D.

Andere geeignete Monomere D enthalten Schwefelsäure- und/oder Sulfatgruppen. Von diesen seien beispielsweise Natriumallylsulfat und Natriummethallylsulfat erwähnt.

Weiterhin geeignet sind Sulfatgruppen enthaltende Monomere D der allgemeinen Formel

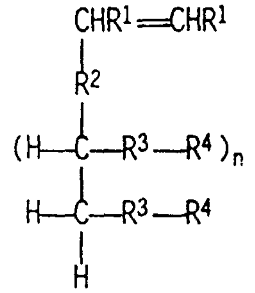

(V)

In der Formel V bedeutet

- $R^1$ Wasserstoff oder den Methylrest,
- $R^2$ einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung,
- $R^3$ -O- oder -NH-,
- $R^4$ Wasserstoff, den Rest $-SO_3^-Na^+$ oder den Rest $-SO_3^-N(R^5)_4^+$, wobei $R^5$ die der in Formel I angegebene Bedeutung hat, und steht
- n für 4 oder 5;

mit der Maßgabe, daß mindestens einer der Substituenten $R^4$ ein Rest $-SO_3^-Na^+$ oder $-SO_3^-N(R^5)_4^+$ ist.

In bevorzugten Monomeren D der Formel V bedeutet

- $R^1$ Wasserstoff,
- $R^2$ einen Alkylenrest mit 1 bis 4 Kohlenstoffatomen, einen Phenylenrest oder eine C-C-Einfachbindung.
- $R^3$ -O- oder -NH-,
- $R^4$ Wasserstoff oder den Rest $-SO_3^-Na^+$ und steht
- n für 4.

Die Monomeren D der Formel V enthalten modifizierte Zuckerreste, die vorzugsweise von Pentosen und insbesondere von Arabinose abgeleitet sind. Die Zuckerreste enthalten mindestens einen der Reste $-O-SO_3^-Na^+$ ("O-Sulfat") oder $-NH-SO_3^-Na^+$ ("N-Sulfat"), bevorzugt benachbart zu dem Rest $R^2$. Sie weisen vorzugsweise 1 bis 4 dieser Reste auf. In einem Zuckerrest können O-Sulfat- und N-Sulfatreste gleichzeitig vorliegen, wobei dann der N-Sulfatrest bevorzugt benachbart zum Rest $R^2$ positioniert ist. Alternativ kann der Zuckerrest aber auch ausschließlich eine Art dieser Reste enthalten, z.B. nur O-Sulfatreste. Jede der genannten Spezies (nur O-Sulfat enthaltende sowie N-Sulfathaltige Reste) ist für sich allein oder zusammen mit der anderen Spezies als Monomer D der Formel V geeignet. Das Mischungsverhältnis beträgt also 0:100 bis 100:0.

Die Herstellung von Monomeren D der Formel V ist in der gleichzeitig anhängigen Patentanmeldung 192 20 369.8 (O.Z. 5195) beschrieben.

### 4.5 Bestimmte Kombinationen aus Monomeren C und D

Anstelle von zwei Monomeren C und D mit den Formeln II und III kann das erfindungsgemäße Polymer auch nur ein Monomer (II + III) enthalten, das die verschiedenen charakteristischen Gruppen in demselben Molekül aufweist. Natürlich kann man statt nur eines Momomers C zwei oder mehr Monomeren C und anstelle nur eines Monomers D zwei oder mehr Monomere D einsetzen. So kann man statt Acrylsäure allein eine Mischung aus Acrylsäure und Natriumacrylat und statt Natrium-4-styrolsulfonat eine Mischung aus Natrium-4-styrolsulfonat und 4-Styrolschwefelsäure verwenden.

Für die erfindungsgemäßen Polymeren hat sich eine Kombination aus Monomeren C und D bewährt, die einerseits Carboxyl- und/oder Carboxylatgruppen und andererseits Sulfonsäure- und/oder Sulfonatgruppen aufweisen. Es gibt unter dem Aspekt der Verträglichkeit hinsichtlich drei mögliche Zweierkombinationen aus den genannten Gruppen, nämlich Carboxyl- und Sulfonsäuregruppen, Carboxyl- und Sulfonatgruppen sowie Carboxylat- und Sulfonatgruppen, und weiterhin zwei Dreierkombinationen, nämlich Carboxyl-, Carboxylat- und Sulfonatgruppen sowie Carboxyl-, Sulfonsäure- und Sulfonatgruppen. Alle diese Kombinationen charakterisieren brauchbare Vertreter der erfindungsgemäßen

Polymeren. Natürlich können auch Monomere enthalten sein, die durch nachträgliche Veränderung von funktionellen Gruppen nach der Polymerisation entstanden sind. So kann man z.B. den Acrylamid-Baustein nachträglich durch Hydrolyse in saurem Medium in den Acrylsäure-Baustein umwandeln. Weiterhin kann man Carboxyl- und Sulfonsäuregruppen durch Neutralisieren (z.B. in Phosphatpuffern) sowie Carbonester- und Sulfosäureestergruppen durch Hydrolyse in Carboxylat- bzw. Sulfonatgruppen überführen.

In der genannten Kombination aus Monomeren C und D kann das molare Verhältnis von Carboxyl- und/oder Carboxylatgruppen zu Sulfonsäure- und/oder Sulfonatgruppen in weiten Grenzen schwanken. Vorteilhaft liegt es jedoch im Bereich von 0.1 bis 10. Bei einem solchen Verhältnis zeigen die Polymeren ausgeprägte bakterienabweisende Eigenschaften. Besonders ausgeprägte zellproliferationsinhibierende Eigenschaften werden zusätzlich erzielt, wenn das genannte Verhältnis 0,2 bis 3, vorteilhaft 0,4 bis 3 und insbesondere 0,4 bis 2 beträgt. Die beschichteten Oberflächen zeigen in bemerkenswerter Weise bakterienabweisende, aber zellproliferationsfördernde Eigenschaften, wenn das besagte molare Verhältnis 2 bis 10, vorteilhaft 3 bis 10 und insbesondere 3 bis 5 beträgt. Zellproliferationsfördernd im Sinne der Erfindung ist ein Polymer dann, wenn die Haftung und Vermehrung von Säugetierzellen verbessert oder jedenfalls weniger stark beeinträchtigt wird als die Haftung und Vermehrung von Bakterien.

### 4.6 Weitere Monomere (Monomere E)

Neben den Monomeren A bis D kann ein oder können mehrere weitere olefinisch ungesättigte Monomere (Monomere E) in dem erfindungsgemäßen Polymer enthalten sein. Von ihrer Art und Menge hängt es ab, ob das Polymer wasserlöslich oder nur quellbar ist. Wenn man das Polymer als Beschichtungsmittel aus wäßrigem Medium aufbringen will, ist die Mitverwendung von hydrophilen, z.B. hydroxylgruppenhaltigen Monomeren gut möglich. Hydroxylgruppen oder Aminogruppen enthaltende Monomere E sind auch für die kovalente Anbindung des erfindungsgemäßen Polymers an Substrate nützlich. Durch orientierende Versuche läßt sich unschwer ermitteln, welche Monomeren E in welchen Mengen in dem erfindungsgemäßen Polymer enthalten sein könne, damit es den jeweils gestellten Anforderungen, z.B. hinsichtlich seiner mechanischer Eigenschaften, entspricht.

Als Monomere E können z.B. verwendet werden (Meth)acrylsäurederivate, wie Ethylacrylat, Isobutylacrylat, 2-Ethylhexylacrylat, 2-Hydroxyethylacrylat, 2-(2'-Hydroxyethoxy)ethylacrylat, 2-Hydroxy-1-methylethylacrylat, 2-N,N-Dimethylaminoethylacrylat, n-Propylmethacrylat, 2-Hydroxyethylmethacrylat, 2-(2'-Hydroxyethoxy)ethylmethacrylat, 2-Hydroxy-1-methylethylmethacrylat, 2-N,N-Dimethylaminoethylmethacrylat, Diethylenglykolmonomethacrylat, (Meth)acrylamid und (Meth)acrylnitril; olefinisch ungesättigte Amine, wie Vinylamin und Allylamin; Vinylketone, wie Vinylethylketon; Olefine, wie 1-Buten, 1-Hexen und 1-Octen; Vinylaromaten, wie Styrol, $\alpha$-Methylstyrol und Vinyltoluol; und Vinylsiloxane.

### 4.7 Herstellung der erfindungsgemäßen Polymeren

Die Polymeren werden in üblicher Weise durch radikalisch initiierte Polymerisation hergestellt, vorteilhaft durch Lösungs- oder Emulsionspolymerisation. Geeignete Lösemittel sind z.B. Ketone, wie Aceton, Methylethylketon und Cyclohexanon; Ether, wie Diethylether, Tetrahydrofuran und Dioxan; Alkohole, wie Methanol, Ethanol, n- und iso-Propanol, n- und iso-Butanol und Cyclohexanol; stark polare Lösemittel, wie Dimethylformamid, Dimethylacetamid und Dimethylsulfoxid; Kohlenwasserstoffe, wie Heptan, Cyclohexan, Benzol und Toluol; Halogenkohlenwasserstoffe, wie Dichlormethan und Trichlormethan; Ester, wie Ethylacetat, Propylacetat und Amylacetat; sowie Nitrile, wie Acetonitril; oder Gemische aus diesen Lösemitteln.

Geeignete Polymerisationsinitiatoren sind z.B. Azonitrile, Alkylperoxide, Acylperoxide, Hydroperoxide, Peroxyketone, Peroxyester und Percarbonate sowie alle üblichen Photoinitiatoren. Die Polymerisation wird thermisch, z.B. durch Erhitzen auf 60 bis 100°C. oder durch Strahlung mit entsprechender Wellenlänge eingeleitet. Nach Beendigung der exothermen Polymerisationsreaktion wird das Polymer in üblicher Weise vom Lösemittel abgetrennt, beispielsweise durch Fällung mittels n-Hexan. Durch Extraktion mit einem geeigneten Lösemittel, z.B. mit Wasser oder wäßrigem Alkohol, können monomere oder oligomere Bestandteile entfernt werden.

Man kann anstelle der Monomeren C und D auch Monomere mit funktionellen Gruppen einsetzen, die nach der Polymerisation in die für die Monomeren C und D charakteristischen funktionellen Gruppen umgewandelt werden. So kann man Carbonestergruppen durch Behandlung mit wäßrigem oder wäßrig-alkoholischem Alkali in Carboxylatgruppen oder aber Carbonester-, Carbonamid- oder Nitrilgruppen durch Hydrolyse in saurem Medium in Carboxylgruppen umwandeln. Weiterhin kann man, wie bereits erwähnt, das Verhältnis von Carboxyl- zu Carboxylatgruppen bzw. von Sulfonsäure- zu Sulfonatgruppen oder Schwefelsäure- zu Sulfatgruppen nach der Polymerisation mit alkalischen oder sauren Reagenzien in erwünschter Weise verändern.

Die Mengenverhältnisse, in denen die Monomeren A bis D eingesetzt werden, können in weiten Grenzen variieren. Vorteilhaft beträgt der Anteil des Monomers A, Maleinsäureanhydrid, 15 bis 50 Mol-%, vorzugsweise 35 bis 50 Mol-%; der Anteil des Monomers B ebenfalls 15 bis 50 Mol-%, vorzugsweise 35 bis 50 Mol-%; der Anteil des Monomers

C 0,5 bis 30 Mol-%, vorzugsweise 0,5 bis 15 Mol-%; und der Anteil des Monomers D ebenfalls 0,5 bis 30 Mol-%, vorzugsweise 0,5 bis 15 Mol-%, Wenn das erfindungsgemäße Polymer weitere Monomere E enthält, beträgt deren Anteil in der Regel 0,5 bis 69 Mol-%, vorzugsweise 0,5 bis 30 Mol-%.

### 4.8 Beschichtung von Substraten mit den erfindungsgemäßen Polymeren

Ein weiterer Gegenstand der Erfindung ist ein Verfahren zum Beschichten von Substraten, insbesondere von Polymersubstraten, bei dem man das erfindungsgemäße Polymer kovalent auf dem Substrat bindet, wozu es reaktiver Gruppen sowohl im erfindungsgemäßen Polymer als auch auf dem Polymersubstrat bedarf. Weiterhin sind Erzeugnisse aus Polymermaterialien, Keramik oder Metall, die ganz oder teilweise mit den erfindungsgemäßen Polymeren beschichtet sind, ein Gegenstand der Erfindung.

Mit dem erfindungsgemäßen Polymer lassen sich die verschiedensten Polymersubstrate beschichten. Geeignete Polymere sind z.B. Polyamide, wie Polyamid-6, Polyamid-12, Polyamid-6,6 und Polyamid-6,12; Polyurethane aus den verschiedensten Diisocyanaten und Polyolen, Polyether- und Polyesterpolyolen; Polyetherblockamide; Polyesterblockamide; Polyetheresteramide; Polyester aus aromatischen Dicarbonsäuren und den verschiedenen aliphatischen oder cycloaliphatischen Diolen; weiterhin Polyvinylchlorid, Polystyrol, Polyacrylate, Polymethacrylate, Polyether, Polycarbonate, Polyolefine, Silicone und Polytetrafluorethylen.

Die genannten Polymeren enthalten häufig Gruppen, wie Carboxyl-, Amino- oder Hydroxylgruppen, die für die kovalente Anbindung des Beschichtungspolymers hilfreich sind. Alternativ lassen sich solche Gruppen auf der Oberfläche des Polymers erzeugen, wofür eine Vielzahl bekannter Methoden zur Verfügung steht. So kann man entsprechende Monomere einpolymerisieren oder Standardpolymere modifizieren, z.B. durch Behandlung mit Säuren oder Basen; Bestrahlung mit UV-Licht, Elektronen- oder Röntgenstrahlen; Behandlung mit Hochfrequenz- oder Mikrowellen-Plasma; oder Beflammen. Weiterhin kann man auch Primer mit reaktiven Gruppen auf Polymere aufbringen, die von Haus aus keine reaktiven Gruppen mitbringen und keiner der genannten oder ähnlichen Behandlungen unterzogen wurden. Geeignete Primer sind z.B. Polyisocyanate, wie Toluylendiisocyanat, Hexamethylendiisocyanat, 4,4'-Diphenylmethandiisocyanat, 4,4'-Dicycloxymethandiisocyanat und Isophorondiisocyanat; Polyamine, wie Ethylendiamin, Triethylendiamin, 1,3-Propylendiamin, 1,4-Butylendiamin und 1,6-Hexamethylendiamin; Polyaldehyde, wie Succindialdehyd, Adipindialdehyd und Terephthaldialdehyd; sowie Polyepoxide, wie Ethylenglykoldiglydidylether und 1,3-Propylenglykoldiglycidylether.

Eine Vorbehandlung mit einem geeigneten Primer ermöglicht auch die kovalente Anbindung des erfindungsgemäßen Beschichtungspolymers auf anorganischen Substraten, wie Keramik oder Metall, z.B. auf Edelstahl.

Es gibt zahlreiche Möglichkeiten zur kovalenten Anbindung des Beschichtungspolymers an ein Polymersubstrat bzw. einen Primer. So können Carbonsäureanhydridgruppen des Beschichtungspolymers mit Aminogruppen auf der Oberfläche des Polymersubstrates reagieren, die z.B. (i) durch $NH_3$-Plasma, (ii) durch Alkalibehandlung eines Polyamids oder Polyurethans, (iii) durch Hydrolyse von einpolymerisierten oder durch Primen mit einem Polyisocyanat eingeführten NCO-Gruppen oder (iv) durch Primen mit einem Alkylendiamin erzeugt worden sein können. Alternativ können z.B. NCO-Gruppen auf dem Substratpolymer, die überschüssige NCO-Gruppen aus der Synthese eines Polyurethans sein oder durch Behandlung eines Polymersubstrates mit Hydroxyl- oder Aminogruppen auf der Oberfläche mit einem (gegebenenfalls blockierten) Polyisocyanat als Primer entstanden sein können, mit Hydroxylgruppen im Beschichtungspolymer reagieren, die z.B. durch Einpolymerisieren eines hydroxylgruppenhaltigen Monomers E in das Molekül eingeführt wurden. Eine dritte Variante besteht in der Verknüpfung eines Polymersubstrates mit Hydroxyl - oder Aminogruppen auf der Oberfläche mit einem Beschichtungspolymer, das einpolymerisierte Amino- oder Hydroxylgruppen enthält, mit Hilfe eines Diepoxids. Weiterhin lassen sich Dicarbonsäuredihalogenide als Primer einsetzen, wenn Polymersubstrat und Beschichtungspolymere COCl-reaktive Gruppen aufweisen. Alle diese Reaktionen sind an sich bekannt, und dem Fachmann werden unschwer weitere Verknüpfungsmechanismen gegenwärtig sein, je nach der Art der Funktionalität der reaktiven Gruppen auf dem Polymersubstrat, im Beschichtungspolymer und gegebenenfalls im Primer.

Primer und Beschichtungspolymer werden zweckmäßig in Lösung auf das Polymersubstrat aufgebracht. Als Lösemittel eignen sich z.B. Wasser; Alkohole, wie Methanol, Ethanol und n-Butanol; Ketone, wie Aceton , Butanon (Methylethylketon) und Cyclohexanon; Ester, wie Methylacetat, Methylpropionat, Ethylacetat und n-Butylacetat; Ether, wie Ethylenglykoldiethylether und Tetrahydrofuran; sowie aromatische Kohlenwasserstoffe, wie Toluol und Xylol. Die Wahl des Lösemittels hängt auch von der Natur des Polymersubstrates ab, das nicht gelöst werden darf, wohl aber angequollen werden soll. Wenn Wasser oder Alkohole als Lösemittel eingesetzt werden, müssen die NCO-Gruppen von als Primer vorgesehenen Polyisocyanaten in üblicher Weise geschützt werden, z.B. mit Methylethylketoxim.

Wenn ein Polymersubstrat vor der Beschichtung mit einem Primer behandelt wird, läßt man dessen Lösung zweckmäßig einige Zeit, z.B. 5 Minuten bis 2 Stunden, auf das Substrat einwirken, gegebenenfalls bei erhöhter Temperatur, z.B. bis zu 60°C und läßt dann das Lösemittel verdampfen, gegebenenfalls und Vakuum und/oder bei erhöhter Temperatur.

Zur Beschichtung des Polymersubstrats eignen sich die bekannten Verfahren, wie Tauchen, Spritzen, Streichen, Rakeln und Spin-Coating. Nach dem Beschichten wird wiederum das Losemittel verdampft. Zur kovalenten Verknüpfung des Beschichtungspolymers mit dem Substrat wird das beschichtete Substrat zweckmäßig noch einige Zeit, z.B. 5 Minuten bis 2 Stunden, auf erhöhter Temperatur gehalten, z.B. auf 50 bis 90°C. Wenn ein blockiertes Isocyanat die Verknüpfung bewirken soll. können entsprechend höhere Temperaturen erforderlich sein.

Beschichtung mit den Polymeren nach der Erfindung macht es möglich, bekannte und bewährte Materialien und Herstellverfahren weiterzuverwenden. Dies ist insbesondere dann wichtig, wenn die mechanischen Eigenschaften der Werkstoffe von hoher Bedeutung sind oder die Fertigungsanlagen nach den bestehenden Herstellverfahren hohe Investitionen erfordern.

### 3.6 Erfindungsgemäße Erzeugnisse

Besondere Vorteile bieten die Polymeren nach der Erfindung bei Erzeugnissen, die in Bewegung an empfindlichen Oberflächen entlanggeführt werden. Das ist z.B. der Fall bei Kathetern und Schläuchen, die die Innenwände von Gefäßen, wie Venen und Arterien, infolge des geringen Reibungswiderstandes schonend beanspruchen.

Die erfindungsgemäß hergestellten Erzeugnisse sind insbesondere zur Verwendung auf den Gebieten der Nahrungs- und Genußmitteltechnik. Wassertechnik, Biotechnik, der Hygienevorsorge und insbesondere der Medizintechnik bestimmt. Beispielsweise lassen sich die hier beschriebenen Polymeren verwenden zur Herstellung von Erzeugnissen. wie Textilien, Geräten, Rohren und Schläuchen, Kondomen, Verpackungen und von medizintechnischen Artikeln, die eine Beschichtung mit den erfindungsgemäßen Polymeren auf Kunststoffen, Keramiken oder Metall als Substrat aufweisen. Medizintechnische Artikel sind beispielsweise Implantate oder Hilfsmittel, wie Drainagen, Führungsdrähte, Kanülen, Intraokularlinsen, Kontaktlinsen, Stents, Gefäßprothesen, Gelenkprothesen, Dialysemembranen und Nahtmaterialien. Bevorzugte Erzeugnisse sind Katheter oder Schläuche zur medizinischen Verwendung.

Die folgenden Bespiele sollen die Erfindung weiter erläutern, nicht jedoch ihren Umfang begrenzen, wie er in den Patentansprüchen dargelegt ist.

### Beispiele

### Messung der Bakterienadhäsion beschichteter Standardfolien über ATP (statisch)

Nach einer Adsorption von Mikrobenzellen an Standardfolien mit Beschichtungen aus dem jeweiligen erfindungsgemäßen Polymer werden die nichtadhärierten Bakterien mit steriler PBS-Pufferlösung weggespült. Aus den an der Folie adhärierenden Bakterien wird in üblicher Weise der Bakterieninhaltsstoff Adenosintriphosphat (ATP) extrahiert und mit einer handelsüblichen Testkombination im bioluminometrischen Test bestimmt. Die Anzahl der gemessenen Lichtimpulse ist proportional zur Zahl der adhärierenden Bakterien. Als Referenzprobe dient eine unbeschichtete Standardfolie. Wird beispielsweise eine Polyamid 12-Folie mit einem erfindungsgemäßen Polymer beschichtet, so dient eine unbeschichtete Polyamid 12-Folie als Referenzprobe. Für die Berechnung der Hemmung der Bakterienadhäsion wird die Adsorption an der Referenzfolie gleich 100 gesetzt. Die Hemmung ist die Differenz aus der Adsorption an der beschichteten und an der unbeschichteten Standardfolie.

### Messung des Reibungswiderstandes

Auf eine Glasscheibe, deren Neigungswinkel gegenüber der Horizontalen stufenlos verstellbar und meßbar ist, wird eine unbeschichtete Standardfolie aufgeklebt. Ein Stück der beschichteten Standardfolie wird mit der beschichteten Seite nach außen in die Spannvorrichtung eines rechteckigen Prüfgewichtes (6 cm x 4 cm, Masse 100 g) eingespannt. Die beschichtete und die unbeschichtete Standardfolie werden tropfnaß befeuchtet, und das Prüfgewicht wird auf die mit der Standardfolie beklebte Glasplatte gesetzt. Der Neigungswinkel der Glasplatte wird bei 0° beginnend stufenlos und ruckfrei mit etwa 20°/min solange erhöht, bis das aufgesetzte Gewicht in Bewegung gerät. Der zu diesem Zeitpunkt eingestellte Winkel wird notiert. Sein Tangens ist der Haftreibungskoeffizient.

### Beispiel 1

In einen 1 l-Fünfhalskolben, der mit Rührer, Rückflußkühler und vier Tropftrichtern versehen ist, werden unter Schutzgas (Stickstoff) 100 ml Dimethylsulfoxid (DMSO) gegeben. Aus 100 ml DMSO und 70 g Maleinsäureanhydrid (MSA) wird eine Lösung (Zulauf 1) hergestellt, die in den Tropftrichter 1 gegeben wird. In den Tropftrichter 2 werden 62 ml Vinylmethylether (VME) gefüllt (Zulauf 2). Tropftrichter 3 enthält eine Lösung (Zulauf 3) aus 7,2 ml Acrylsäure (AS), 19.6 g Natriumstyrolsulfonat (NaSS) und 70 ml DMSO. In den Tropftrichter 4 werden 1,5 g Azoisobutyronitril in 20 ml DMSO gegeben (Zulauf 4). Aus dem Tropftrichter 1 werden 8 ml Zulauf 1 und aus den Tropftrichtern 2, 3 und 4 werden

jeweils 5 ml Zulauf 2, 3 und 4 in den Kolben dosiert. Danach wird die Mischung auf 70°C erhitzt. Nach weiteren 10 Minuten werden Zulauf 1, 2 und 3 innerhalb von 3,5 Stunden und wird Zulauf 4 innerhalb von 4 Stunden zudosiert. Nach beendeter Zugabe wird die Reaktionslösung noch weitere 4 Stunden auf 70°C gehalten. Das Quaterpolymer wird durch Einrühren in 2 l n-Hexan ausgefällt und mit Wasser gewaschen. Die NMR-Analyse des Quaterpolymers ergibt eine Zusammensetzung von

| | |
|---|---|
| MSA | 43,2 Mol-% |
| VME | 45,1 Mol-% |
| AS | 6,6 Mol-% |
| NaSS | 5,1 Mol-% |

Das Molverhältnis von COOH bzw $COO^-$ zu $SO_3^-$ beträgt 1.3.

Zur Messung der Bakterienadhäsion wird eine Polyamid 12-Folie mit dem Quaterpolymer beschichtet. Dazu wird ein Lösung des Quaterpolymers in Dimethylsulfoxid (20 g/l) hergestellt. Diese Lösung sprüht man auf die Polyamid 12-Folie und läßt das Lösemittel verdampfen. Die Messung der Bakterienadsorption mit Klebsiella pneumoniae ergab eine Verminderung gegenüber der unbeschichteten Polyamid 12-Folie um 92 %.

Zur Messung des Reibungskoeffizienten wurde die, wie beschrieben, mit dem Quaterpolymer beschichtete Folie auf das Prüfgewicht geklebt. Die Messung des Reibungswiderstandes gegenüber einer unbeschichteten Polyamid 12-Folie ergab einen Haftreibungskoeffizienten von 0,05. Der Haftreibungskoeffizient von zwei unbeschichteten Polyamid 12-Folien beträgt 0,42.

**Beispiel 2**

Es wird verfahren wie im Beispiel 1, der Zulauf 3 ist jedoch eine Lösung aus 9 ml Acrylsäure, (AS), 14,4 g Natriumstyrolsulfonat (NaSS) und 70 ml DMSO. Die NMR-Analyse des Quaterpolymers ergibt eine Zusammensetzung von

| | |
|---|---|
| MSA | 42,9 Mol-% |
| VME | 44,9 Mol-% |
| AS | 7,5 Mol-% |
| NaSS | 4,7 Mol-% |

Das Molverhältnis von COOH bzw $COO^-$ zu $SO_3^-$ beträgt 1,6.

Die Messung der Bakterienadhäsion mit Klebsiella pneumoniae ergibt eine Verminderung gegenüber einer unbeschichteten Polyamid 12-Folie um 95%.

Der Haftreibungskoeffizient beträgt 0,06.

**Beispiel 3**

Eine Polyamid 12 Folie (9 cm x 6 cm) wird unter Schutzgas (Argon) bei Raumtemperatur für 1 Stunde in eine 1-gewichtsprozentige Lösung von Diphenylmethandiisocyanat in Ethylmethylketon getaucht. Die Folie wird entnommen und in einem Trockenschrank bei 60°C für die Dauer von 30 Minuten getrocknet. Die getrocknete Folie wird dann bei Raumtemperatur für 4 Sekunden in eine 2-gewichtsprozentige Lösung des Polymers aus Beispiel 1 in Dimethylsulfoxid getaucht. Dann wird die Folie zunächst 1 Stunde im Trockenschrank bei 60°C und darauf 5 Stunden bei 70°C und einem Druck von 1 mbar getrocknet. Danach wird die Folie 3 Stunden in Wasser von 25°C gelegt und 24 Stunden bei 60°C getrocknet.

Die Hemmung der Adsorption von Klebsiella pneumoniae beträgt ebenso wie die der anderen zuvor genannten Bakterienstamme >92%. Der Haftreibungskoeffizient beträgt 0,05.

Ähnliche Ergebnisse erzielt man, wenn man in analoger Weise das Polymer des Beispiels 2 kovalent an eine Polyamid 12-Folie bindet.

**Patentansprüche**

1. Copolymer, welches als Monomere

   (A) Maleinsäureanhydrid (Monomer A);
   (B) mindestens einen Vinylalkylether, dessen Alkylrest 1 bis 6 Kohlenstoffatome umfaßt (Monomer B);
   (C) mindestens ein Carboxyl- und/oder Carboxylatgruppen enthaltendes Vinylmonomer (Monomer C) sowie
   (D) mindestens ein Schwefelsäure-, Sulfat-, Sulfonsäure- und/oder Sulfonatgruppen enthaltendes Vinylmonomer (Monomer D)

einpolymerisiert enthält.

**2.** Polymer nach Anspruch 1, dadurch gekennzeichnet, daß es Sulfat- oder Sulfonatgruppen und Carboxyl- oder Carboxylatgruppen enthält.

**3.** Polymer nach Anspruch 2, dadurch gekennzeichnet, daß die Gegenionen zu den genannten anionischen Gruppen Natriumionen oder Ammoniumionen sind.

**4.** Polymer nach einem der Ansprüche 1 bis 3, dadurch gekennzeichnet, daß das Monomer B Vinylmethylether ist.

**5.** Polymer nach einem der Ansprüche 1 bis 4, dadurch gekennzeichnet, daß das Monomere C der allgemeinen Formel

$$(C_nH_{2n-q-x})(COOR^1)_x \qquad (I)$$

entspricht, in der

<u>n</u> jeweils unabhängig für eine ganze Zahl von 2 bis einschließlich 6;
<u>x</u> jeweils unabhängig für 1 oder 2;
<u>q</u> jeweils unabhängig für 0 oder 2 steht; und
$R^1$ jeweils unabhängig -H, ein Alkalimetallion oder ein Ammoniumion bedeutet.

**6.** Polymer nach einem der Ansprüche 1 bis 4, dadurch gekennzeichnet, daß das Monomer C der allgemeinen Formel

$$(C_6H_{6-b-c-d})[(C_nH_{2n-1-q-x})(COOR^1)_x]_bR^3{}_c(OH)_d \qquad (II)$$

entspricht, in der

$R^1$, <u>n</u>, <u>q</u> und <u>x</u> wie in der Formel I definiert sind;
$R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2-CH_2-N^+(CH_3)_3$, $-PO(O^-)O-CH_2-CH_2-N^+(CH_3)_3$, $-OP(O^-)OCH_2-CH_2-N^+(CH_3)_3$ oder ein Salz, insbesondere ein Alkalisalz, der genannten Gruppen bedeutet;
<u>b</u> für 1, 2, oder 3 steht;
<u>c</u> für 0, 1, 2, oder 3 steht; und
<u>d</u> für 0, 1, 2, oder 3 steht;

mit der Maßgabe, daß $b + c + d \leq 6$, vorteilhaft $\leq 4$ ist.

**7.** Polymer nach einem der Ansprüche 1 bis 6, dadurch gekennzeichnet, daß das Monomer D der allgemeinen Formel

$$(C_nH_{2n-q-x})(SO_3R^1)_x, \qquad (III)$$

entspricht, in der

<u>n</u>, <u>x</u>, <u>q</u> und $R^1$ die für die Formel I angegebenen Bedeutungen haben.

**8.** Polymer nach einem der Ansprüche 1 bis 6, dadurch gekennzeichnet, daß das Monomer D der allgemeinen Formel

$$(C_6H_{6-b-c-d})[(C_nH_{2n-1-q-x})(SO_3R^1)_x]_bR^3{}_c(OH)_d \qquad (IV)$$

entspricht, in der $R^1$, <u>n</u>, <u>q</u> und <u>x</u> sowie $R^3$, <u>b</u>, <u>c</u> und <u>d</u> wie in der Formel II definiert sind.

**9.** Polymer nach einem der Ansprüche 1 bis 6, dadurch gekennzeichnet, daß das Monomer D der allgemeinen Formel

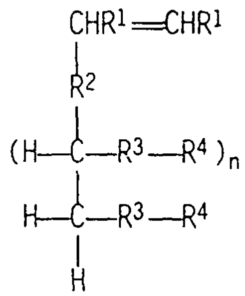

(V)

entspricht, in der

- $R^1$ Wasserstoff oder den Methylrest,
- $R^2$ einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung.
- $R^3$ -O- oder -NH-,
- $R^4$ Wasserstoff, den Rest $-SO_3$-$Na^+$ oder den Rest $-SO_3^-N(R^5)_4^+$ bedeutet, wobei die Reste $R^5$ jeweils unabhängig Wasserstoff oder einen organischen Rest bezeichnen, und
- n für 4 oder 5 steht;

mit der Maßgabe, daß mindestens einer der Substituenten $R^4$ ein Rest $-SO_3^-Na^+$ oder $-SO_3^-N(R^5)_4^+$ ist.

**10.** Polymer nach einem der Ansprüche 1 bis 8, dadurch gekennzeichnet, daß das Polymer einerseits Carboxyl- und/oder Carboxylatgruppen und andererseits Sulfonsäure- und/oder Sulfonatgruppen aufweist.

**11.** Polymer nach Anspruch 10, dadurch gekennzeichnet, daß das Verhältnis von Carboxyl- und/oder Carboxylatgruppen zu Sulfonsäure und bzw. oder Sulfonatgruppen 0,1 bis 10 beträgt.

**12.** Polymer nach Anspruch 10, dadurch gekennzeichnet, daß das Verhältnis von Carboxyl- und/oder Carboxylatgruppen zu Sulfonsäure und bzw. oder Sulfonatgruppen 0,2 bis 3 beträgt.

**13.** Polymer nach Anspruch 10, dadurch gekennzeichnet, daß das Verhältnis von Carboxyl- und/oder Carboxylatgruppen zu Sulfonsäure und bzw. oder Sulfonatgruppen 2 bis 10 beträgt.

**14.** Verfahren zur Herstellung der Polymeren nach den Ansprüchen 1 bis 13, dadurch gekennzeichnet, daß man die Monomeren A, B, C und D radikalisch polymerisiert.

**15.** Verfahren zum Beschichten von Substraten, dadurch gekennzeichnet, daß man ein Polymer nach einem der Ansprüche 1 bis 13 mittels reaktiver Gruppen sowohl im Polymer als auch auf dem Substrat kovalent auf dem Substrat bindet.

**16.** Verfahren nach Anspruch 15, dadurch gekennzeichnet, daß man die reaktiven Gruppen auf dem Substrat durch Behandeln mit einem Primer mit reaktionsfähigen Gruppen schafft.

**17.** Verwendung der Polymeren nach den Ansprüchen 1 bis 13 für die Herstellung von Erzeugnissen zur Verwendung auf den Gebieten der Nahrungs- und Genußmitteltechnik, Wassertechnik und Biotechnik, der Hygienevorsorge und insbesondere der Medizintechnik.

**18.** Erzeugnisse, die ganz oder teilweise mit Polymeren nach den Ansprüchen 1 bis 10 beschichtet sind, zur Verwendung auf den Gebieten der Nahrungs- und Genußmitteltechnik, Wassertechnik und Biotechnik, der Hygienevorsorge und insbesondere der Medizintechnik.

**19.** Erzeugnisse nach Anspruch 18, dadurch gekennzeichnet, daß die Erzeugnisse Katheter oder Schläuche zur medizinischen Verwendung sind.